# EUROPEAN PATENT SPECIFICATION

(11) **EP 3 498 175 B1**
(45) Date of publication and mention of the grant of the patent: **06.08.2025**
(21) Application number: 17839657.8
(22) Date of filing: 03.07.2017
(51) Int. Cl.: A61P 37/08, A61B 10/00, A61B 5/00, A61K 9/00, A61K 9/70

(54) **MICRONEEDLE PATCH AND MANUFACTURING METHOD THEREFOR**
MIKRONADELPFLASTER UND HERSTELLUNGSVERFAHREN DAFÜR
PATCH À MICRO-AIGUILLES ET SON PROCÉDÉ DE FABRICATION

(30) Priority: 12.08.2016 KR 20160103053
(43) Date of publication of application: 19.06.2019
(73) Proprietor: PROLAGEN Co., Ltd., Seoul 08375 (KR); Raphas Co., Ltd., Seoul 03920 (KR)
(72) Inventor: LEE, Kwang Hoon, Seoul 07339 (KR); SHIN, Jung U, Seoul 03709 (KR); KIM, Ji Hye, Pohang-si Gyeongsangbuk-do 37667 (KR); KIM, Seo Hyeong, Seoul 03764 (KR); KIM, Hong Kee, Gunpo-si Gyeonggi-do 15815 (KR); KIM, Jung Dong, Seoul 03951 (KR); LEE, Yang Gi, Seoul 03918 (KR); KIM, Seong Jin, Seoul 03721 (KR); JEONG, Do Hyeon, Seoul 03904 (KR)
(74) Representative: Fabry, Bernd
(86) International application number: PCT/KR2017/007030
(87) International publication number: WO 2018/030639

(56) References cited:
- EP-A1- 2 119 469
- WO-A1-2014/182932
- WO-A2-2004/047794
- JP-A- 2014 156 433
- JP-A- 2014 156 433
- KR-A- 20110 046 205
- KR-A- 20140 051 648
- KR-A- 20150 138 647
- KR-A- 20150 138 647
- KR-B1- 101 254 240
- KR-B1- 101 618 523
- US-A1- 2010 030 100
- US-A1- 2016 058 377

## Description

### [Technical Field]

The present disclosure relates to a micro-needle patch, a method for manufacturing the micro-needle patch, and a method for diagnosing an allergy disease using the micro-needle patch. More particularly, the present disclosure relates to a micro-needle patch for diagnosing and treating an allergy disease which can easily and effectively inject an allergen into a dermal layer of an entity, a method for producing the micro-needle patch, and a method for diagnosing an allergy disease using the micro-needle patch

### [Background Art]

A living organism produces antibodies and lymphocytes that react specifically to an antigen as a heterologous substance. Then, when the living organism again contacts the heterologous substance, the antibodies and lymphocytes react with the antigen to cause various immune reactions. This immune reaction is an important defense mechanism for self-preservation of the living organism.

However, the immune reaction may be caused by a number of substances that are not harmful to living organisms, such as pollen, drugs, food, animal hair, house dust mites, food additives, and may be harmful to the living organisms. Such immune hypersensitivity reaction is called allergies. Allergies may lead to diseases such as bronchial asthma, allergic rhinitis, urticaria, edema, atopic dermatitis, drug allergy, and anaphylactic shock.

In order to identify an allergy-inducing substance (usually allergen) of a patient suspected of having an allergy, a skin prick test and a multiple allergen simultaneous test (MAST) are executed. In the skin prick test, a suspected allergen is selected and injected into the skin tissue to assess a skin reaction such as swelling and rash. The MAST may collect blood from patients and perform simultaneous tests on dozens of allergens.

However, the skin prick test causes a significant pain for the patient when the needle is inserted through the skin. Further, in the skin prick test, the diagnosis results may vary according to the injection action by the diagnostician and the like and may be inaccurate. In the MAST, blood is drawn from the patient and the blood is separately tested in the laboratory. The accuracy of the diagnostic results may be high, but the cost of testing is high and it takes a long time to check the results.

To overcome these limitations, a patch test was introduced to assess the skin reaction by attaching a patch with a selected allergen directly to the skin (*See* prior patent document 1). However, according to the patch test, it takes about 48 to 96 hours for the allergen to be injected through the epidermal layer into the dermis layer and then for allergic reactions to occur. Thus, there arises a problem that when the patient has such a patch attached thereto, he/she experiences inconvenience in daily life as in a body washing.

Thus, there is a growing need for an allergen injection method or device that can effectively inject the allergen into the dermal layer without causing pain or discomfort to the patient.

### [Prior Art Document]

Japanese patent publication JP 2014 156433 A discloses a microneedle patch according to the preamble of claim 1.

### [Patent Literature]

1. Korean Patent No. 10-0849757 (Title of the invention: Patch for screening the sensitivity of a subject with respect to allergen and use thereof)

### [Disclosure]

### [Technical Problem]

In order to solve the conventional problems, a purpose of the present disclosure is to provide a micro-needle patch for simply diagnosing or treating an allergy disease, in which the patch may easily and effectively inject an allergen into a dermis of an entity, and may be structurally simple.

Further, another purpose of the present disclosure is to provide a method for manufacturing such a micro-needle patch and a method for diagnosing an allergy disease using such a micro-needle patch.

Purposes of the present disclosure are not limited to the purposes mentioned above. Other purposes not mentioned may be clearly understood by those skilled in the art from following descriptions.

### [Technical Solution]

The invention which embodies the technical solution is defined in appended claim 1. Further advantageous aspects of the invention are defined in the dependent claims.

A method of manufacturing the inventive micro-needle patch is defined in claim 7.

### [Technical Effect]

The micro-needle patch in accordance with the present disclosure may inject the allergen easily and effectively into the dermis of the entity, thereby to allow diagnosing or treating the allergy disease easily and efficiently without causing great pain to the patient. Further, controlling the type and content of the allergen contained in the micro-needle patch may allow diagnosing or treating allergy diseases accurately. Moreover, using the micro-needle patch containing the allergen at a high concentration may help diagnose or treat the allergy disease more effectively.

### [Description of Drawings]

FIG. 1 is a top view of each of micro-needle patches and each of allergen injection mechanisms thereof according to various embodiments of the present disclosure.
FIG. 2 is a top view illustrating each of various forms of a biodegradable micro-needle patch of the present disclosure.
FIG. 3 is a flowchart illustrating a method for fabricating the micro-needle patch illustrated in FIGS. 2(A) and 2(B).
FIG. 4 is a flowchart illustrating a method for fabricating the micro-needle patch illustrated in FIG. 2(C).
FIG. 5 is a flowchart illustrating a method for fabricating the micro-needle patch illustrated in FIG. 2(D).
FIG. 6 is a photograph of a micro-needle containing 10 µg of house dust mite allergen of *D. farinae* species.
FIG. 7 is a graph illustrating a change in a concentration of IgE (immunoglobulin E) in serum and a change in absorbance at 450 nm wavelength based on increasing allergen content.
FIG. 8 is a graph illustrating a change in a concentration of allergen contained in the patch after the micro-needle patch has been subjected to a radiation-based sterilization process.
FIG. 9 is a graph illustrating a change in concentration of allergen over time.
FIG. 10 illustrates photographs of a micro-needle biodegradation procedure over time after attaching the micro-needle patch in accordance with the present disclosure to the skin of an entity.
FIG. 11 illustrates photographs regarding activities of an MHC2 marker and D. *farinae* allergen in a dermis.
FIG. 12 illustrates photographs for comparing effects of attaching the micro-needle patch of the present disclosure and a conventional patch to the skin of the entity, respectively.
FIG. 13 is a flowchart illustrating a method for diagnosing an allergic disease using the micro-needle patch of the present disclosure.
FIG. 14 is a flowchart detailing an operation of checking an allergic reaction in FIG. 13.

### [Best Mode]

Other advantages and features of the present disclosure and methods of achieving the other advantages and features will become apparent with reference to embodiments as described below in detail with reference to the accompanying drawings. However, the present disclosure is not limited to the embodiments disclosed below, but may be embodied in many different forms. Those embodiments only allow the present disclosure to be complete. Those embodiments allow the skilled person to the art to completely understand the present disclosure. A scope of the present disclosure is not defined by the embodiments but is defined by attached claims.

Shapes, sizes, ratios, angles, numbers and the like disclosed in the drawings for illustrating the embodiments of the present disclosure are illustrative, and thus the present disclosure is not limited to the illustrated shapes, sizes, ratios, angles, numbers and the like. Further, descriptions and details of well-known operations and elements are omitted for simplicity of the description and not to unnecessarily obscure aspects of the present disclosure.

When the terms "comprises", "comprising", "includes", and "including" are used in this specification, other portions may be added unless "only" is used. The singular forms of the elements include the plural unless the context clearly dictates otherwise. In interpreting the constituent elements, it is construed to include the error range even if there is no separate description.

Like reference numerals refer to like elements throughout the specification. The sizes and thicknesses of the individual components shown in the drawings are illustrated for convenience of explanation and the present disclosure is not necessarily limited to the size and thickness of the components illustrated. Features of various embodiments of the present disclosure may be combined with or coupled to each other, either partially or entirely. As will be appreciated by those skilled in the art, the features of various embodiments of the present disclosure may be associated with each other in a variety of schemes. The embodiment may be practicable independently of one another or in conjunction with one another.

As used herein, a term "allergy" refers to all forms of immunologic hypersensitivity reactions to a specific allergen which may be detrimental to a host manifesting the reaction, and phenomena resulting therefrom. Recently, IgE antibody-mediated immediate hypersensitivity reaction type I to the allergen present in an external environment to the entity has been narrowly referred to as allergic reaction. As used herein, the allergic reaction is not limited thereto. As used herein, the allergic reaction should be interpreted as a broad allergic reaction that includes hypersensitive reaction types I to IV for all allergens present inside and outside the entity.

Therefore, as used herein, the term "allergen" refers to all kinds of substances that may cause the immune hypersensitivity reaction in the entity. The allergen may not be limited to one of an exogenous allergen and an endogenous allergen (self-contained allergen), but may also refer to a substance that induces an immune hypersensitivity reaction in the entity. Thus, as used herein, the allergen may be any one selected from a group consisting of house dust mites, fungi, pollen, animal hair, metals, plastics, rubbers, drugs, microorganisms, foods, protein-based or choline-based substances such as self-contained proteins, or a combination thereof. Hereinafter, the allergen is assumed to be a house dust mite of *D. farinae* species for convenience of illustration. However, it should be noted that the allergen in the present disclosure is not limited thereto.

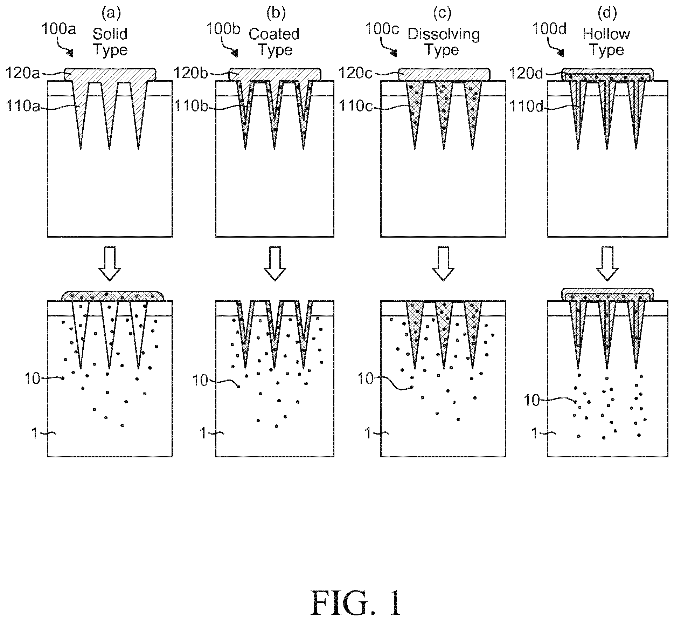

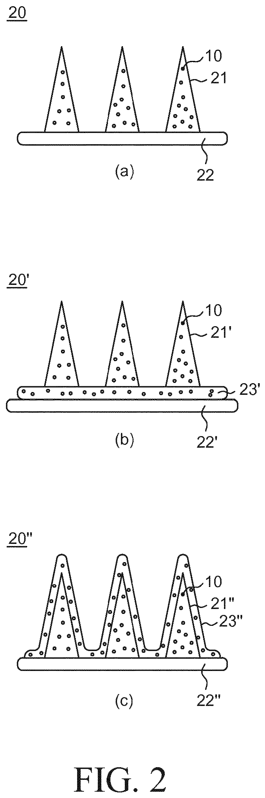

Hereinafter, micro-needle patches according to various embodiments of the present disclosure will be described with reference to FIGS. 1 and 2 attached hereto.

FIG. 1 is a top view of each of micro-needle patches and each of allergen injection mechanisms thereof according to various embodiments of the present disclosure. FIG. 2 is a top view illustrating each of various forms of a biodegradable micro-needle patch of the present disclosure.

Referring to FIG. 1, each of micro-needle patches 100a, 100b, 100c and 100d according to the present disclosure includes each of a plurality of micro-needles 110a, 110b, 110c and 110d configured to inject an allergen 10 into the entity, and each of a plurality of pads 120a, 120b, 120c and 120d to support each of the plurality of micro-needles 110a, 110b, 110c and 110d.

Further, each of the micro-needle patches according to various embodiments may further include each of textile weave or nonwoven sheets disposed on each of the pads 120a, 120b, 120c and 120d. Each adhesive layer may be formed on each of the pads 120a, 120b, 120c and 120d to prevent each micro-needle patch from moving when each patch is attached to the skin of the entity.

FIG. 1(A) illustrates a solid type micro-needle patch 100a and a method of injecting an allergen 10 into a skin 1 of an entity using a solid type micro-needle 110a. The solid type micro-needle 110a creates a channel in the skin 1 to define a passage of the allergen 10. By attaching a separate patch containing the allergen 10 on the channel, the allergen 10 can be injected into the skin 1 of the entity.

FIG. 1(B) illustrates a coated type micro-needle patch 100b and a method for injecting the allergen 10 into the skin 1 of the entity using a micro-needle 110b of this type. The coated type micro-needle 110b may be applied with a composition containing the allergen 10 on the above-described solid type micro-needle 110a. Thus, when the patch is attached to the entity, the applied allergen 10 can be injected into the skin 1.

FIG. 1(C) illustrates a biodegradable type or dissolvable type micro-needle patch 100c and a method for injecting the allergen 10 into the skin 1 of the entity using a micro-needle 110c of this type. The biodegradable or dissolvable type micro-needle 110c is biodegraded or dissolved in the skin 1 of the entity and allows the allergen 10 to be injected into the skin 1. The biodegradable or dissolvable type micro-needle patch 100c will be described in more detail below with reference to FIG. 2.

FIG. 1(D) illustrates a hollow-type micro-needle patch 100d and a method for injecting the allergen 10 into the skin 1 of the entity using a micro-needle 110d of this type. The hollow-type micro-needle 110d may provide a passage through which the allergen 10 contained in the pad 120d passes. The allergen 10 may be injected through this passage into the skin 1.

The solid type micro-needle patch 100a, the coated type micro-needle patch 100b, the dissolvable type micro-needle patch 100c and the hollow type micro-needle patch 100d have different injection schemes of the allergen 10. However, the solid type micro-needle patch 100a, the coated type micro-needle patch 100b, the dissolvable type micro-needle patch 100c and the hollow type micro-needle patch 100d may be equally able to inject the allergen 10 into the skin 1 of the entity to cause the allergic reaction therein. Thus, the micro-needle patch according to the present disclosure may include all of the types in FIG. 1(A) to FIG. 1(B) as described above.

Referring to FIG. 2, the dissolvable type micro-needle patch 100c as illustrated in FIG. 1(c) may be formed according to various variation examples.

Each of the dissolvable type micro-needle patches 20, 20' and 20" includes each of a plurality of dissolvable type micro-needles 21, 21' and 21", and each of a plurality of pads 22, 22' and 22" supporting each of the plurality of dissolvable type micro-needles 21, 21' and 21". Furthermore, each of the dissolvable type micro-needle patches 20, 20' and 20" according to the present disclosure may further include each of allergen-containing layers 23' and 23".

Each of the plurality of dissolvable type micro-needles 21, 21' and 21" contains the allergen 10 and is composed of a material which may be dissolved *in vivo* by body fluids, enzymes or microorganisms. In this connection, the biodegradation or dissolving may be interpreted to include both dissolution of the allergen 10 *in vivo* by the body fluid and biodegradation thereof by enzymes *in vivo.*

In this regard, it is preferred that each of the micro-needles 21, 21' and 21" is made of a dissolved substance that minimizes a side effect and effectively transfers a drug when inserted into the entity. This kind of the substance may be preferably hyaluronic acid (HA).

However, the present disclosure is not limited thereto. Each of the dissolvable type micro-needles 21, 21' and 21" is composed of a bio-derived soluble substance such as chitosan, collagen, gelatin, myristic acid, behenic acid, cellulose or derivatives thereof, maltose, dextrin, glucosamine, heparin, polylysine, other animal wax, or the like. In another example, each of the dissolvable type micro-needles 21, 21' and 21" is composed of a biocompatible substance such as polyvinylpyrrolidone (PVP), polyethylene glycol (PEG), polyvinyl alcohol (PVA), hydroxypropyl cellulose (HPC), hydroxyethyl cellulose (HEC), hydroxypropyl methylcellulose (HPMC), polylactic acid, polyglycolic acid, and the like. Further, it is needless to say that each of the dissolvable type micro-needles 21, 21' and 21" is composed of any biodegradable substance known in the art.

Each of the pads 22, 22' and 22" has a plate-like structure connected to each of the micro-needles 21, 21' and 21" to support each of the plurality of micro-needles 21, 21' and 21". Each of the pads 22, 22' and 22" is preferably integrated with each of a plurality of the micro-needles 21, 21' and 21". However, the present disclosure is not limited thereto. Each of the pads 22, 22' and 22" may be formed as each of separate members separated from each of the plurality of micro-needles 21, 21', and 21".

Each of the pads 22, 22' and 22" may be made of a separate known substance that is not biodegradable. Alternatively, each of the pads 22, 22' and 22" may be made of the same dissolved substance as that of each of the micro-needles 21, 21', and 21" as described above. A structure and composition of each of the pad 22, 22', and 22" may not be limited to specific structure and composition as long as each of the pad 22, 22', and 22" provides a supporting force which may support each of the plurality of micro-needles 21, 21' and 21", and allows each of the micro-needle 21, 21' and 21" to inject the allergen 10 into the entity when each of the micro-needle patches 20, 20' and 20" according to the present disclosure is attached to the skin of the entity.

Each of the allergen-containing layers 23' and 23" contains allergen 10 of the same kind of the allergen 10 contained in each of the micro-needle 21' and 21". Each of the allergen-containing layers 23' and 23" has a technical significance of increasing a content of the allergen per patch.

As illustrated in FIG. 2(B), the allergen-containing layer 23' may be formed to be disposed between the micro-needles 21' and the pad 22' and to cover the pad 22'. Alternatively, the allergen-containing layer 23' may be applied only between the plurality of micro-needles 21' and may not be disposed below the micro-needles 21' such that the micro-needles 21' and the pad 22'may directly contact each other.

In various embodiments, the allergen-containing layer 23" may be formed to cover the micro-needles 21" on the plurality of micro-needles 21"as illustrated in FIG. 2(C).

Each of the micro-needles 21, 21' and 21" of the micro-needle patches 20, 20', and 20" according to the present disclosure preferably has a length of from 200 µm to 350 µm so that the allergen 10 can be injected at a depth of about 250 µm into the human skin. However, the above-defined length range is an example. The present disclosure is not necessarily limited thereto. It is needless to say that the length of each of the micro-needles 21, 21', and 21" may be determined based on a pre-set injection point of the allergen 10.

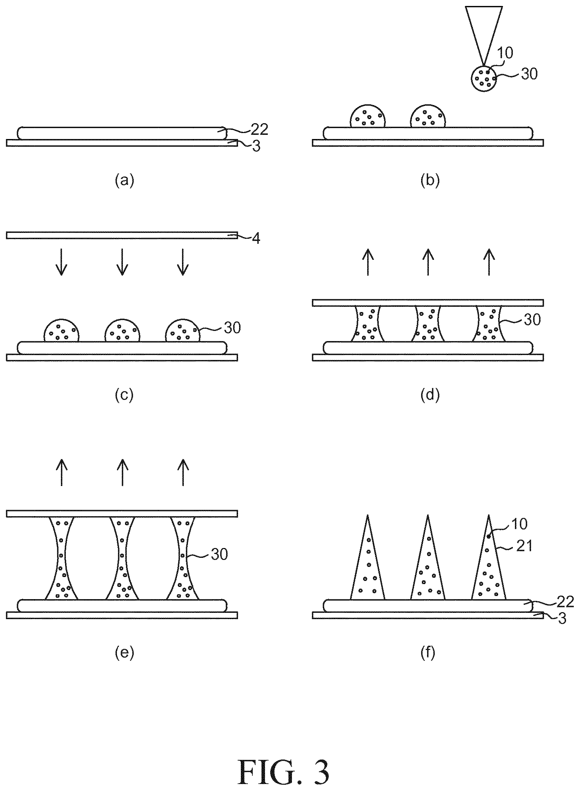

Hereinafter, with reference to FIGS. 3 to 5, a method for manufacturing the various dissolvable type micro-needle patches 20, 20', and 20" of FIG. 2 as described above will be described.

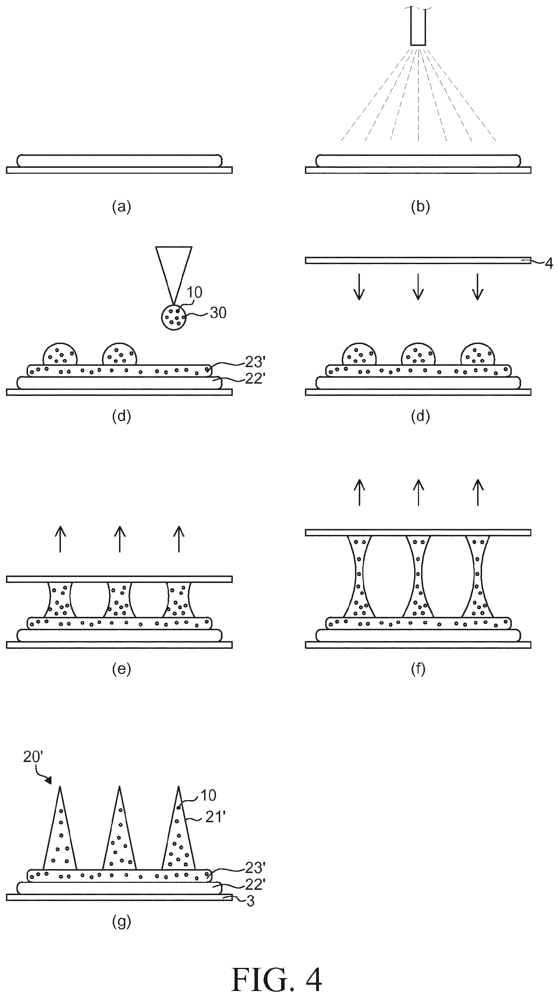

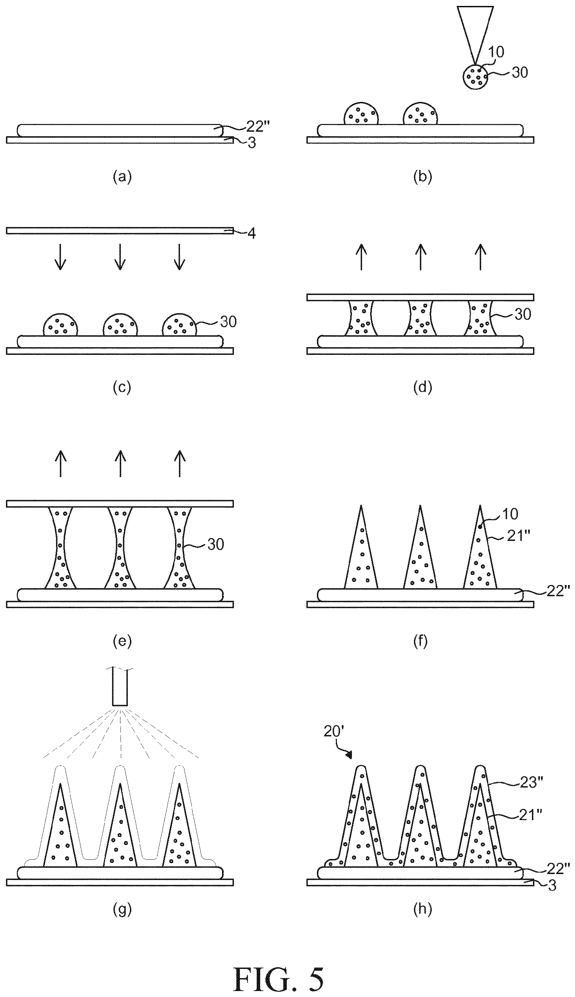

FIG. 3 is a flowchart illustrating a method for fabricating the micro-needle patch 20 illustrated in FIG. 2(A). FIG. 4 is a flowchart illustrating a method for fabricating the micro-needle patch 20' illustrated in FIG. 2(B). FIG. 5 is a flowchart illustrating a method for fabricating the micro-needle patch 20" illustrated in FIG. 2(C).

Referring to FIG. 3, a method for fabricating the micro-needle patch 20 according to the present disclosure includes forming a pad 22, spotting a viscous composition 30 containing allergen 10, stretching and solidifying the spotted viscous composition 30, and forming micro-needles 21 by cutting the solidified composition 30.

Forming the pad 22 may be performed by applying a composition on a first substrate 3 and then drying the composition as illustrated in FIG. 3(A). At this time, blowing may be carried out to facilitate the drying.

In this connection, the first substrate 3 is in a form of a flat plate as illustrated and its material is not particularly limited. However, since the first substrate 3 is used for manufacturing a micro-needle patch used for a medical purpose, it is preferable that the first substrate 3 is made of a material which is harmless to the human body.

The composition means a substance having a viscosity to allow forming of a structure of the pad on the first substrate 3. The composition may include a substance defining the pad as described above, and may preferably include hyaluronic acid. Further, the composition that forms the pad may contain an allergen. In this connection, the allergen is preferably the same as the allergen 10 contained in the viscous composition 30 forming the micro-needle 21 to be described later.

Next, spotting the viscous composition 30 containing the allergen 10 is performed. In this connection, the viscous composition 30 containing the allergen 10 may include the above-mentioned dissolved substance that is injected into the entity and is biodegraded *in vivo.*

The spotting step may be performed at a pre-set interval using a known dispenser.

When the viscous composition 30 containing the allergen 10 is spotted, a second substrate 4 is brought into contact with the plurality of viscous composition 30 as illustrated in FIG. 3(C).

The second substrate 4 may be in a form of a flat plate like the first substrate 3. In an alternative, the second substrate 4 may have a pre-determined protruding portion (a portion corresponding to the spot) to allow the micro-needle 21 to be formed.

After the second substrate 4 and a portion of the viscous composition spot 30 touch each other, relatively spacing by a vertical distance between the first substrate 3 and the second substrate 4 may allow the viscous composition spot 30 to be stretched as illustrated in FIG. 3(D) and FIG. 3(E). Then, the stretched viscous composition 30 is solidified. Although the stretching usually precedes the solidification, initiations of the stretching and solidification may be performed simultaneously. An order of initiations of the stretching and solidification may not be limited to a specific order.

In the stretching and solidifying procedure, blowing may be applied to the stretched viscous composition 30.

Finally, as illustrated in FIG. 3(F), the solidified viscous composition 30 is cut to form a plurality of micro-needles 21.

In this connection, a length of the micro-needle 21 as formed, a diameter of upper and lower ends thereof, and an aspect ratio thereof may vary depending on fabrication parameters such as a viscosity of the viscous composition 30 containing the allergen 10, a relative movement speed between the first substrate 3 and second substrate 4, and a blowing intensity. Each of the above-described operations may be appropriately designed and changed in accordance with a target specification of the micro-needle 21. Further, the method of manufacturing the micro-needle patch 20 under controlling a strength and shape of the micro-needle 21 is not limited to the above-defined method but may be performed by referring to the technique disclosed in Korean Patent Application Publication No. 10-2014-0051648.

Although the micro-needle may be manufactured by spotting the viscous composition only on the first substrate as illustrated in FIG. 3, the present disclosure may not be limited thereto. The viscous composition may be spotted or applied on both the first substrate and the second substrate. Then, the viscous composition spotted on the first substrate may be brought into contact with the viscous composition of the second substrate, thereby forming the micro-needles. In this connection, the viscous composition spotted or applied on the second substrate may preferably contain the same component as the viscous composition spotted or applied on the first substrate.

In one example, the viscous composition may be spotted at a plurality of spaced points on the first substrate. The viscous composition may be spotted at the corresponding points on the second substrate. Then, the first and second substrates may be aligned so that the viscous composition spots on the first substrate and the second substrate correspond to each other and contact each other. The viscous compositions may be stretched to form the micro-needles.

Further, in another example, the viscous composition may be spotted at a plurality of spaced points on the first substrate. Then, the viscous composition may be uniformly applied to the second substrate. Then, the micro-needle may be produced by contacting the first and second substrates and stretching the viscous compositions.

In methods of producing the micro-needle patches 20' and 20" of FIGS. 4 and 5, an operation of forming each of the allergen-containing layers 23' and 23" is added to the method of producing the micro-needle patch 20 of FIG. 3.

An operation of forming the allergen-containing layer 23' in FIG. 4(B) is performed immediately after the operation of forming the pad 22' such that the allergen-containing layer 23' is formed to cover the pad 22'. Further, an operation of forming the allergen-containing layer 23" in FIG. 5(G) is performed after the operation of cutting the solidified micro-needle 21" such that the allergen-containing layer 23" is formed to cover the plurality of micro-needles 21". Although not illustrated, in various embodiments, after the micro-needles 21' are formed, the allergen-containing layer 23' may be applied between the micro-needles 21' in FIG. 4.

Other operations in FIG. 4 and FIG. 5 correspond to the operations of the manufacturing method of the micro-needle patch 20 in FIG. 3 as described above. Thus, redundant descriptions therebetween may be omitted.

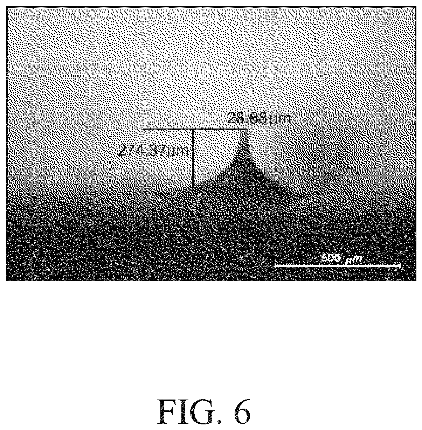

FIG. 6 illustrates an enlarged view (FIG. 6(A)) of the micro-needle patch prepared according to one of the methods as described above and an enlarged view (FIG. 6(B)) of one micro needle of the micro-needle patch.

Specifically, the micro-needle patch in FIG. 6(A) was prepared to contain 10 µg of allergen containing house dust mite of *D. farinae* species and hyaluronic acid. Referring to FIG. 6(B), it may be checked that the micro-needle has 274.37 µm of an overall length and has 28.88 µm of a top diameter.

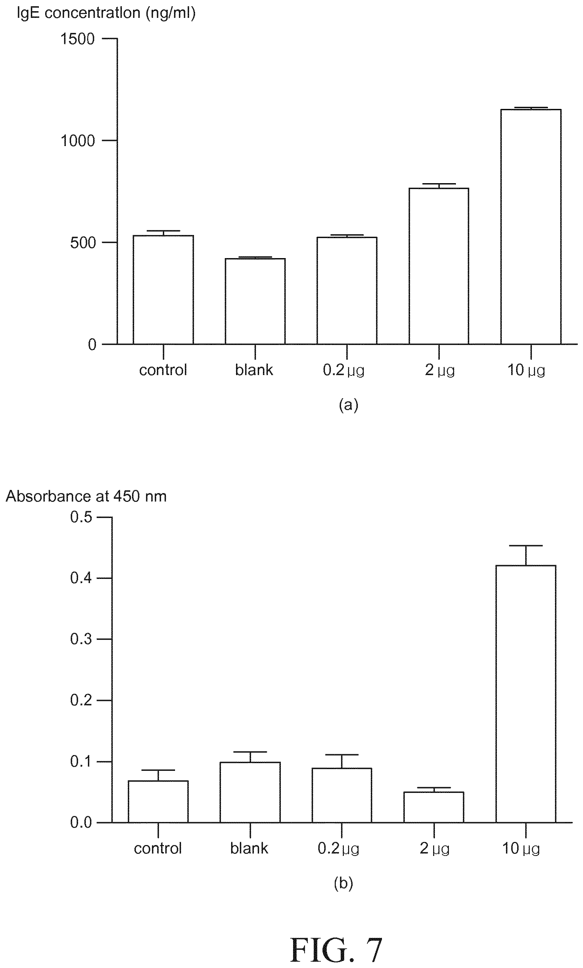

Hereinafter, the micro-needle patch according to the present disclosure will be described with reference to experimental results or photographs of FIGS. 7 to 12.

In FIG. 7, in order to check the activity of the allergen-mediated immune reaction, the change of an IgE concentration in serum and the change of absorbance at 450 nm wavelength based on increasing allergen contents per patch were measured and the measurements were illustrated in the graph.

Specifically, FIG. 7(A) illustrates a measurement of a concentration of IgE antibodies in the serum of the entity in 4 weeks after the micro-needle patches prepared with increasing the content of the allergen per patch to 0.2, 2, and 10 µg were attached to the entity. The allergen used in the experiment was house dust mite of *D. farinae* species. This allergen is known to cause a typical IgE antibody-mediated immediate allergic reaction.

When a control, the patch containing 0.2 µg of the allergen and the patch containing 2 µg of the allergen were attached to the entity, the IgE antibody concentration in the serum of the subject slightly increased from about 500 ng/ml to about 700 ng/ml. To the contrary, when increasing the content of the allergen per patch to 10 µg, the IgE antibody concentration in the serum was significantly increased to about 1200 ng/ml. When the content of allergen per patch increased 10 times from 0.2 µg to 2 µg, the IgE antibody concentration in the serum increased by about 200 ng/ml. When increasing the content of allergen per patch by 5 times from 2 µg to 10 µg, the concentration of IgE antibody in the serum increased by about 500 ng/ml. This value indicates the concentration of IgE antibody in the serum increased at least twice as much as that in 0.2 µg of allergen content per patch. It may be confirmed that increasing the content of allergen per patch to 10 µg or larger is important to cause an immune reaction by allergen delivery.

Furthermore, FIG. 7(B) illustrates the concentration change of the IgE antibody specific to the *D. farinae* house dust mite allergen in the serum of the entity after 4 weeks (as the result of measurement of the absorbance at 450 nm wavelength) after the micro-needle patches prepared with increasing the content of allergen per patch to 0.2 µg, 2 µg, and 10 µg respectively were attached to the entity.

When a control, the patch containing 0.2 µg of the allergen and the patch containing 2 µg of the allergen were attached to the entity, the concentration change of the IgE antibody specific to the *D. farinae* house dust mite allergen in the serum of the entity was slight. To the contrary, when increasing the content of the allergen per patch to 10 µg, the concentration change of the IgE antibody specific to the *D. farinae* house dust mite allergen in the serum of the entity was great. In this connection, the absorbance at the 450 nm wavelength increased greatly. The absorbance at the 450 nm wavelength when the content of allergen per patch was set to 10 µg increased by at least 5 times than the absorbance at the 450 nm wavelength when the allergen content of per patch was set to 2 µg. Thus, it may be confirmed that increasing the content of allergen per patch to 10 µg or larger is important to cause an immune reaction by allergen delivery. Using the micro-needle patch containing allergen at a high concentration of 10 µg or greater for the diagnosis or treatment of the allergic disease may allow diagnosing and treating the allergic diseases more effectively in a shorter time compared to the conventional allergy diagnosis and treatment method.

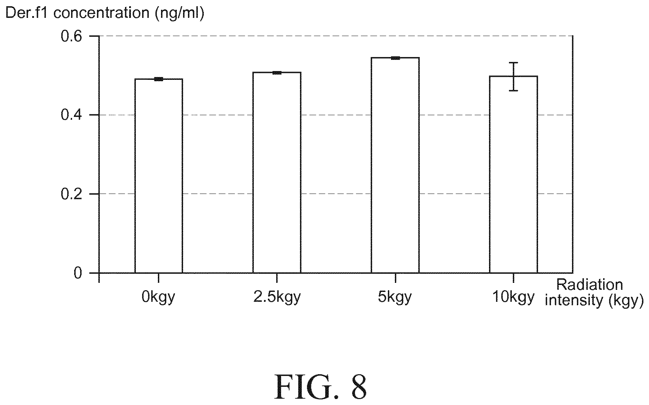

FIG. 8 is a graph illustrating the concentration change of the allergen contained in the patch after the radiation sterilization procedure has been applied to the prepared micro-needle patch.

In order to prevent problems such as bacterial infection that may occur when the micro-needle patch according to the present disclosure is attached to the human body and is used for diagnosing or treating an allergic disease, the above manufactured micro-needle patch was subjected to the radiation sterilization procedure.

We confirmed that even though the micro-needle patch was sterilized while increasing the radiation intensity to 2.5 kgy, 5 kgy, and 10 kgy, there was little concentration change per patch of the house dust mite allergen of *D. farinae* species in the present patch regardless of the radiation intensity, which was not the case in the control patch.

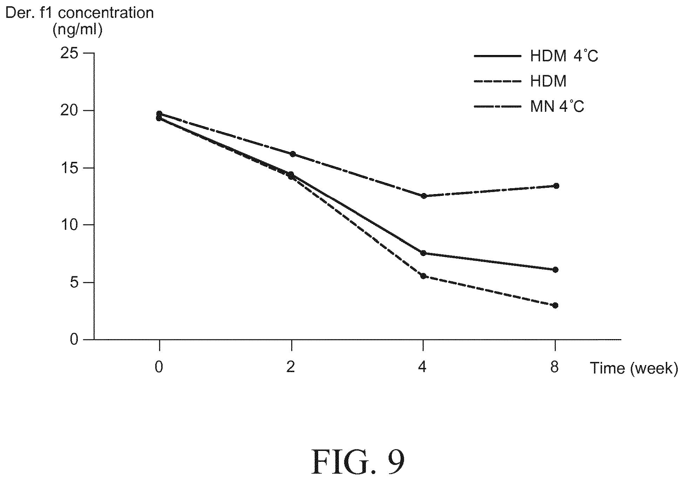

FIG. 9 is a graph illustrating the concentration change of the house dust mite allergen of *D. farinae* species over time. Specifically, the concentration change of allergen left at a room temperature, the concentration change of allergen left at 4°C, and the concentration change of allergen contained in the micro-needle patch according to the present disclosure were measured. The measurements were compared in the graph.

We confirmed that a rate at which the allergen content decreased over time when the allergen was contained in the micro-needle patch according to the present disclosure was lower than a rate at which the allergen content decreased over time when the allergen was contained in a conventional patch. In this connection, the allergen content above 60% of the initial allergen content remained in the patch at a period of 4 weeks after the patch was produced.

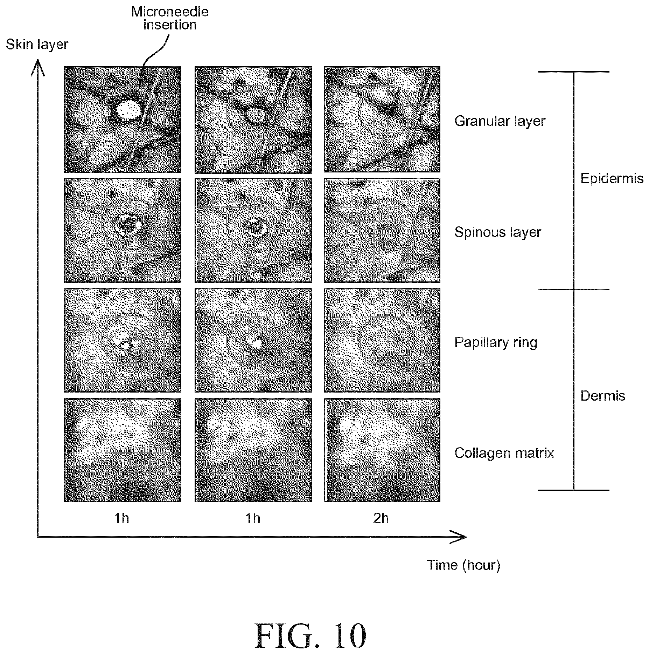

FIG. 10 is a photograph of micro-needle biodegradation or dissolving procedure over time after attaching the micro-needle patch according to the present disclosure to the skin of the entity.

Whether the micro-needle was inserted into the skin when the micro-needle patch according to the present disclosure was attached to the skin of the human body was checked based on a depth of skin using tomography. Then, the micro-needle biodegradation procedure was checked over time lapses of 1 hour, 1 hour and 30 minutes, and 2 hours. It may be confirmed that upon the attachment of the micro-needle patch to the skin, the micro-needle may be inserted to a granular layer and a spinous layer constituting the skin epidermis, and to a papillary layer below a boundary between the epidermis and the dermis.

Further, we checked that the biodegradation of the inserted micro-needle gradually progressed over time. We checked that the biodegradation had been completed and the needle disappeared at 2 hours after the patch attachment. Therefore, when the allergen was injected into the entity using a dissolvable type micro-needle patch according to the present disclosure, approximately at least 90% of the dissolvable type micro-needle inserted within the skin had been dissolved approximately in 2 or 3 hours after the attachment. Thus, the allergen can be injected easily and effectively in a short time into the dermis of the entity.

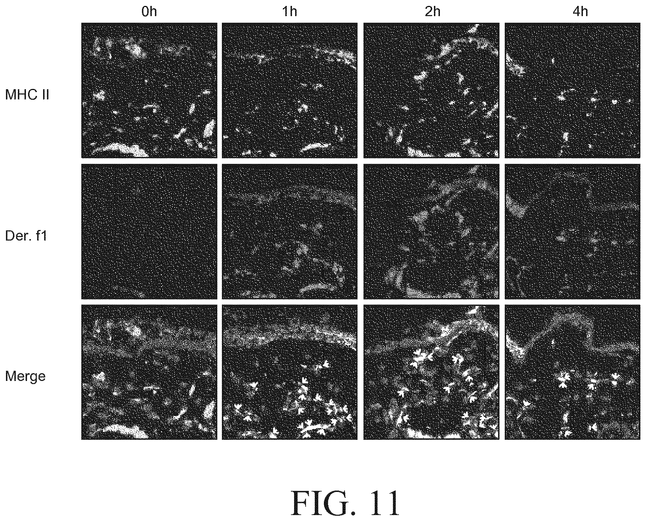

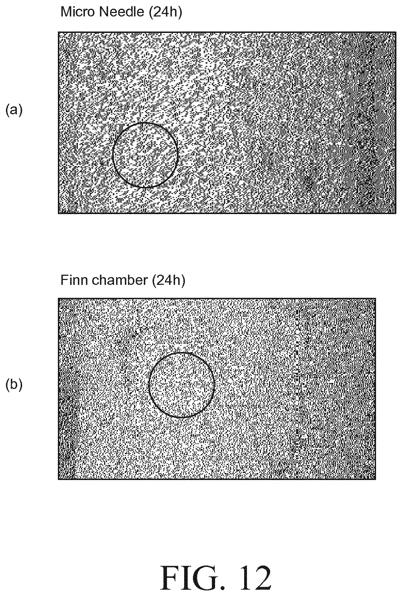

The biodegradation of the micro-needle allows the allergen contained in the micro-needle to be injected into the epidermis and the dermis. In this connection, to check whether the allergen is actually injected into the body and causes the allergic reaction in the human body, a reference will be made to FIG. 11 and FIG. 12 below.

FIG. 11 illustrates photographs regarding activities of an MHC2 marker and *D. farinae* allergen in a dermis.

A distribution of dendritic cells in the skin dermis was checked using MHC2 markers.

After the attachment of the micro-needle patch to the skin, the house dust mite allergen of *D. farinae* species was detected in the skin. We checked, from the photograph taken in 4 hours after the attachment of the patch to the skin that the allergen has been actually delivered to the skin. Furthermore, when considering a combination of the result of measuring the activity of dendritic cells checked by the MHC2 markers and the result of measuring the activity of house dust mite allergen of *D. farinae* species, we confirmed that the allergen injected point and the dendritic cell activation point correspond to each other, such that the allergen as injected causes the allergen reaction.

FIG. 12 illustrates photographs for comparing effects of attaching the micro-needle patch of the present disclosure and a conventional patch to the skin of the entity, respectively.

The subjects were those who did not show an allergic reaction in a conventional attached-type allergy diagnostic patch (finn chamber). The allergic reaction was observed when attaching the micro-needle patch according to the present disclosure to the subjects. No change in the skin color was observed at the skin surface when attaching the conventional allergy diagnostic patch (finn chamber) to the subjects. To the contrary, when attaching the micro-needle patch according to the present disclosure to the subjects, we confirmed via the skin color change to the red that the allergic reaction has occurred.

As a result, the micro-needle patch according to the present disclosure can easily and effectively inject the allergen into the dermis of the entity. We confirmed that the present patch can diagnose or treat an allergic disease simply and efficiently without causing great pain to the patient.

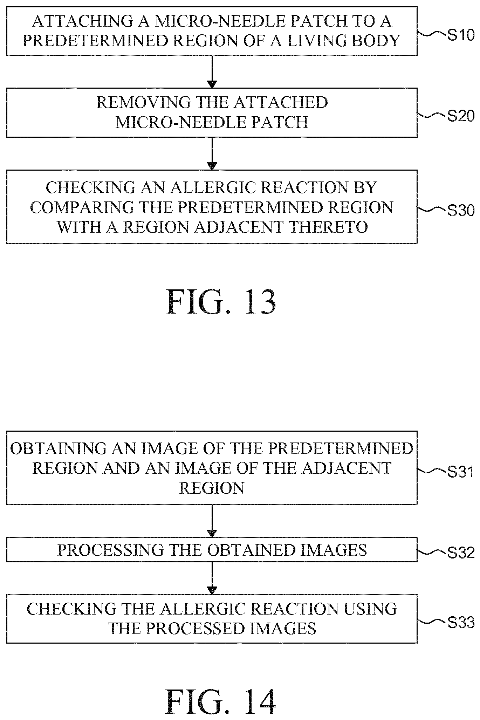

Hereinafter, referring to FIG. 13 and FIG. 14, a method of diagnosing an allergic disease using a micro-needle patch according to the present disclosure will be described.

FIG. 13 is a flowchart illustrating a method for diagnosing an allergic disease using a micro-needle patch according to the present disclosure. FIG. 14 is a flowchart detailing an operation of checking an allergic reaction in FIG. 13.

The method of diagnosing an allergic disease according to the present disclosure may include attaching the micro-needle patch to a pre-set region of the living body in step S10; after a pre-set time amount has elapsed, removing the attached micro-needle patch in step S20; and checking an allergic reaction by comparing the pre-set region with an adjacent region to the pre-set region.

In step S10 of attaching the micro-needle patch, the point at which the patch is attached may be selected as a point at which the patch is easily attached and for which diagnosis is easy. An example of the point may be a point on an arm.

In step S20, in which, after a pre-set time amount has elapsed, the attached micro-needle patch is removed, the pre-set time amount may be adjusted depending on the type of the micro-needle patch. For example, when the dissolvable type micro-needle patch according to the present disclosure is used in the diagnosis of the allergic disease, the patch may be removed 2 hours after the micro-needle is inserted into the skin. At this time, the needle may be almost biodegraded.

Step 30, in which the allergic reaction is checked by comparing the pre-set region and the adjacent region thereto may include obtaining images of the pre-set region and the region adjacent to the pre-set region, respectively in step S31, processing the obtained images in step S32, and checking the allergic reaction via the processed images in step S33.

Usually, at a location to which the micro-needle patch attaches and where the allergic reaction occurs, a skin color becomes red due to swelling and rash. The images of the pre-set region and the adjacent region thereto may be obtained respectively S31 in order to check whether the entity has the allergic reaction to the injected allergen by comparing the redness of the point where the allergic reaction occurred and of the point where no allergic reaction occurred.

This step S31 may be performed, for example, by photographing each of the points.

Step S32 of processing the obtained images may include overlapping the image of the pre-set region and the image of the adjacent region thereto, and offsetting common portions with each other in the overlapped images to check only a difference between the two images.

However, the method of processing the images may not be limited to this method, but may be performed by applying various conventional image processing methods.

Finally, the method determines whether the entity has the allergic disease to the injected allergen by checking the allergic reaction via the processed images in step S33.

While the embodiments of the present disclosure have been described in detail with reference to the accompanying drawings, the present disclosure may not be necessarily limited to these embodiments, and various modifications may be made without departing from the technical idea of the present disclosure. Accordingly, the embodiments disclosed in the present disclosure are intended to be illustrative rather than limiting. Such embodiments do not limit the scope of the technical idea according to the present disclosure. Therefore, it should be understood that the above-described embodiments are illustrative in all aspects and not restrictive. The scope of protection under the present disclosure shall be interpreted in accordance with the following claims.

## Claims

1. A micro-needle patch (20, 20' and 20") comprising:
a plurality of micro-needles (21, 21' and 21") containing an allergen (10) constructed to inject an allergen (10) into an entity; and
a pad (22, 22', and 22') for supporting the plurality of micro-needles (21, 21' and 21"), wherein each micro-needle (21, 21' and 21") is biodegradable, wherein the allergen (10) is contained in the micro-needle (21, 21' and 21") and the micro-needles (21, 21' and 21") are configured such that the allergenis injected into the entity when the micro-needle degrades, **characterized in that** the allergen (10) is contained in a range of 5 µg to 10 µg content per patch and the allergen content of the patch remains at 60% or greater of initial allergen content for a period of 4 weeks to 8 weeks after the patch is produced,
wherein the patch further includes one or both of allergen-containing layers (23' and 23") containing the same type of allergen (10) as the allergen (10) contained in the micro-needles (21' and 21"), and the allergen-containing layer (23') is disposed between the micro-needles (21') and the pad (22') and to cover the pad (22') or the allergen-containing layer (23") covers an outer face of the micro-needles (21").

2. The micro-needle patch (20, 20' and 20") of claim 1, wherein each micro-needle (21, 21' and 21") contains hyaluronic acid.

3. The micro-needle patch (20, 20' and 20") of claim 1, wherein the allergen (10) includes one or a combination of at least two selected from a group consisting of house dust mite, fungus, pollen, animal hair, metal, plastic, rubber, drug, microorganism and food.

4. The micro-needle patch (20, 20' and 20") of claim 3, wherein the house dust mite is of *D. farinae* species.

5. The micro-needle patch (20, 20' and 20") of claim 1, configured such that at least 90% of the micro-needles (21, 21' and 21") are dissolved in 3 hours after the needles are inserted into the entity.

6. The micro-needle patch (20, 20' and 20") of claim 1, wherein a length of each of the micro-needles (21, 21' and 21") is in a range of from 200 µm inclusive to 350 µm inclusive.

7. A method for manufacturing the micro-needle patch (20, 20' and 20") according to one of claims 1 to 6, the method comprising:
spotting a viscous composition containing an allergen at a plurality of spaced points on a first substrate;
contacting a second substrate with the plurality of viscous composition spots on the first substrate;
stretching the viscous composition by spacing between the first substrate and the second substrate vertically;
solidifying the stretched viscous composition; and
forming a plurality of micro-needles by cutting the solidified viscous composition,
wherein the method further comprises applying, onto the formed micro-needle, a composition containing allergen of the same kind as the allergen contained on the spots.

## Patentansprüche

1. Ein Mikronadelpflaster (20, 20' und 20"), umfassend :
eine Vielzahl von Mikronadeln (21, 21' und 21"), die ein Allergen (10) enthalten und dazu konstruiert sind, ein Allergen (10) in ein Subjekt zu injizieren; und
ein Pad (22, 22' und 22') zur Unterstützung der Vielzahl von Mikronadeln (21, 21' und 21"), wobei jede Mikronadel (21, 21' und 21") biologisch abbaubar ist, wobei das Allergen (10) in der Mikronadel (21, 21' und 21") enthalten ist und die Mikronadeln (21, 21' und 21") so konfiguriert sind, dass das Allergen in das Subjekt injiziert wird, wenn die Mikronadel abgebaut wird.
**dadurch gekennzeichnet, dass** das Allergen (10) in einem Bereich von 5 µg bis 10 µg pro Pflaster enthalten ist und der Allergengehalt des Pflasters für einen Zeitraum von 4 Wochen bis 8 Wochen nach der Herstellung des Pflasters bei 60 % oder mehr des ursprünglichen Allergengehalts beibehält,
wobei das Pflaster ferner eine oder beide allergenhaltigen Schichten (23' und 23") umfasst, die denselben Typ von Allergen (10) enthalten wie das in den Mikronadeln (21' und 21") enthaltene Allergen (10), und
die allergenhaltige Schicht (23') zwischen den Mikronadeln (21') und dem Pad (22') angeordnet ist und das Pad (22') abdeckt oder die allergenhaltige Schicht (23") eine Außenseite der Mikronadeln (21") abdeckt.

2. Das Mikronadelpflaster (20, 20' und 20") nach Anspruch 1, wobei jede Mikronadel (21, 21' und 21") Hyaluronsäure enthält.

3. Das Mikronadelpflaster (20, 20' und 20") nach Anspruch 1, wobei das Allergen (10) eines oder eine Kombination aus mindestens zwei Allergenen umfasst, die aus einer Gruppe ausgewählt sind, die aus Hausstaubmilben, Pilzen, Pollen, Tierhaaren, Metall, Kunststoff, Gummi, Arzneimitteln, Mikroorganismen und Nahru ngsmitteln besteht.

4. Das Mikronadelpflaster (20, 20' und 20") nach Anspruch 3, wobei die Hausstaubmilbe zur Art D. farinae gehört.

5. Das Mikronadelpflaster (20, 20' und 20") nach Anspruch 1 ist so konfiguriert, dass sich mindestens 90 % der Mikronadeln (21, 21' und 21") innerhalb von 3 Stunden, nach dem Einführen der Nadeln in das Subjekt, auflösen,.

6. Das Mikronadelpflaster (20, 20' und 20") nach Anspruch 1, wobei die Länge jeder der Mikronadeln (21, 21' und 21") in einem Bereich von einschließlich 200 µm bis einschließlich 350 µm liegt.

7. Verfahren zur Herstellung des Mikronadelpflasters (20, 20' und 20") nach einem der Ansprüche 1 bis 6, wobei das Verfahren umfasst:
Aufbringen einer viskosen Zusammensetzung, die ein Allergen enthält, an mehrere beabstandete Punkte auf einem ersten Substrat;
Inkontaktbringen eines zweiten Substrats mit den mehreren Punkten der viskosen Zusammensetzung auf dem ersten Substrat;
Dehnen der viskosen Zusammensetzung durch vertikales Auseinanderziehen des ersten und zweiten Substrats;
Verfestigen der gedehnten viskosen Zusammensetzung; und
Bilden einer Vielzahl von Mikronadeln durch Schneiden der verfestigten viskosen Zusammensetzung, wobei das Verfahren ferner das Aufbringen einer Zusammensetzung, die ein Allergen derselben Art wie das auf den Punkten enthaltene Allergen enthält, auf die geformten Mikronadeln umfasst.

## Revendications

1. Patch à micro-aiguille (20, 20' et 20") comprenant :
une pluralité de micro-aiguilles (21, 21' et 21") contenant un allergène (10) construites pour injecter un allergène (10) dans une entité ; et
un tampon (22, 22' et 22") pour soutenir la pluralité de micro-aiguilles (21, 21' et 21"),
dans laquelle chaque micro-aiguille (21, 21' et 21") est biodégradable, dans laquelle l'allergène (10) est contenu dans la micro-aiguille (21, 21' et 21") et les micro-aiguilles (21, 21' et 21") sont configurées de manière à ce que l'allergène soit injecté dans l'entité lorsque la micro-aiguille se dégrade,
**caractérisé par le fait que** l'allergène (10) est contenu dans une fourchette de 5 µg à 10 µg par patch et que la teneur en allergène du patch reste à 60 % ou plus de la teneur initiale en allergène pendant une période de 4 semaines à 8 semaines après la production du patch,
dans lequel le patch comprend en outre une ou les deux couches contenant des allergènes (23' et 23") contenant le même type d'allergène (10) que l'allergène (10) contenu dans les micro-aiguilles (21' et 21"), et
la couche contenant l'allergène (23') est disposée entre les micro-aiguilles (21') et le tampon (22') et recouvre le tampon (22') ou la couche contenant l'allergène (23") recouvre une face externe des micro-aiguilles (21").

2. Patch à micro-aiguilles (20, 20' et 20") de la revendication 1, dans lequel chaque micro-aiguille (21, 21' et 21") contient de l'acide hyaluronique.

3. Patch à micro-aiguille (20, 20' et 20") de la revendication 1, dans lequel l'allergène (10) comprend un ou une combinaison d'au moins deux allergènes choisis dans un groupe constitué d'acariens, de champignons, de pollen, de poils d'animaux, de métal, de plastique, de caoutchouc, de médicaments, de micro-organismes et d'aliments.

4. Patch à micro-aiguilles (20, 20' et 20") de la revendication 3, dans lequel l'acarien est de l'espèce D. farinae.

5. Patch à micro-aiguilles (20, 20' et 20") de la revendication 1, configuré de telle sorte qu'au moins 90% des micro-aiguilles (21, 21' et 21") sont dissoutes dans les 3 heures suivant l'insertion des aiguilles dans l'entité.

6. Patch de micro-aiguilles (20, 20' et 20") de la revendication 1, dans lequel la longueur de chacune des micro-aiguilles (21, 21' et 21") est comprise entre 200 µm inclus et 350 µm inclus.

7. Procédé de fabrication du patch à micro-aiguilles (20, 20' et 20") selon l'une des revendications 1 à 6, le procédé comprenant :
l'application d'une composition visqueuse contenant un allergène en plusieurs points espacés sur un premier substrat ;
la mise en contact d'un second substrat avec la pluralité de points de composition visqueuse sur le premier substrat ;
étirer la composition visqueuse en espaçant verticalement le premier substrat et le second substrat ;
solidifier la composition visqueuse étirée ; et
former une pluralité de micro-aiguilles en coupant la composition visqueuse solidifiée,
la méthode comprend en outre l'application, sur la micro-aiguille formée, d'une composition contenant des allergènes du même type que les allergènes contenus sur les taches.
